# EUROPÄISCHE PATENTSCHRIFT

(11) **EP 2 030 598 B1**
(45) Veröffentlichungstag und Bekanntmachung des Hinweises auf die Patenterteilung: **11.12.2013**
(21) Anmeldenummer: 07115544.4
(22) Anmeldetag: 03.09.2007
(51) Int. Cl.: A61F 9/009, A61F 9/01

(54) **Auf ein Auge aufsetzbare Vorrichtung**
Device attachable to an eye
Dispositif pouvant être installé sur un oeil

(43) Veröffentlichungstag der Anmeldung: 04.03.2009
(73) Patentinhaber: Schwind eye-tech-solutions GmbH & Co. KG, 63801 Kleinostheim (DE)
(72) Erfinder: Graener, Heinrich, 06120 Halle (DE); Lange, Jens, 06688 Grosskorbetha (DE); Seifert, Gerhard, 95615 Marktredwitz (DE); Fankhauser, Franz, 3084 Wabern (CH); Hollerbach, Thomas, 63808 Haibach-Dörrmorsbach (DE); Magnago, Thomas, 63762 Wenigumstadt (DE)
(74) Vertreter: Hofstetter, Schurack & Partner

(56) Entgegenhaltungen:
- EP-A- 0 627 207
- WO-A-2006/121066
- DE-A1- 3 612 287
- US-A1- 2003 158 543

## Beschreibung

Die vorliegende Erfindung betrifft eine auf ein Auge aufsetzbare Vorrichtung gemäß dem Oberbegriff des Patentanspruchs 1. Diese Vorrichtung soll ein Hilfsmittel bei lasergestützten refraktiv-chirurgischen Eingriffen sein.

Refraktiv-chirurgische Eingriffe, die mit Hilfe eines Lasers, üblicherweise eines Femtosekundenlasers, durchgeführt werden (z.B. das Schneiden eines Flaps) erfordern die Positionierung des Laserfokus in wohl definierten Tiefen unterhalb der Homhautoberfläche. Die Augenoberfläche ist gekrümmt, was zur Folge hat, dass die Phasenfronten des einfallenden Lichtes verformt werden, wodurch eine genaue Positionierung des Lasersfokus erheblich erschwert wird.

Man behilft sich bisher damit, die Augenoberfläche zu aplanieren, z.B. durch Drücken einer Glasplatte auf die Augenoberfläche selbige flach zu drücken. Hierbei wird das Auge mechanisch stark belastet, und dies ist für die Patienten unangenehm. Die mechanischen Eigenschaften sind bei flach gedrücktem Auge auch nicht vorhersehbar, wodurch die Schnittgenauigkeit bei dem Eingriff eingeschränkt wird.

Die WO 2006/121066 A1 offenbart eine auf ein Auge aufsetzbare Vorrichtung mit den Merkmalen des Oberbegriffs des Anspruchs 1. Aus der EP 0 627 207 A1 ist ebenfalls eine Vorrichtung zur Laserbehandlung eines Auges bekannt. Die Vorrichtung umfasst eine Kühlvorrichtung zur Kühlung des zu behandelnden Abschnitts der Hornhaut des Auges. Zudem ist eine Vorrichtung zur Abgabe eines flüssigen Medikaments auf die Homhautoberfläche ausgebildet.

Es ist Aufgabe der Erfindung, einen Weg aufzuzeigen, wie ein refraktiv-chirurgischer Eingriff mit Hilfe eines Lasers so durchgeführt werden kann, dass der Laserfokus besonders präzise platziert werden kann.

Die Aufgabe wird durch eine Vorrichtung mit den Merkmalen gemäß Patentanspruch 1 gelöst. Zur Lösung der Aufgabe gehört auch ein bei einem Eingriff im Auge einsetzbares System gemäß Patentanspruch 10.

Die erfindungsgemäße, auf ein Auge aufsetzbare Vorrichtung weist einen Grundkörper auf, der eine für zumindestens einen Wellenlängenbereich (nämlich den eines bei Ablationen verwendeten Laserstrahls) transparente Platte trägt. Der Grundkörper hat einen elastischen Fußbereich, der ein Aufsetzen der Vorrichtung auf die Homhautoberfläche eines Auges ermöglicht derart, dass dann zwischen Glasplatte und Homhautoberfläche ein Aufnahmeraum für eine Flüssigkeit ausgebildet ist. An dem Grundkörper ist ferner zumindest ein Einlass für das Einbringen von Flüssigkeit in den Aufnahmeraum (bei auf das Auge aufgesetzter Vorrichtung) bereitgestellt. Zur Steuerung der Flüssigkeitsmenge in dem Aufnahmeraum, ist vorgesehen dass die Platte in variable Stellungen verbringbar ist, denen ein unterschiedlich großer Aufnahmeraum entspricht.

Bei Aufsetzen der Vorrichtung auf das Auge und Füllen des Aufnahmeraums mit einer Flüssigkeit, die einen Brechungsindex hat, der im Bereich des Brechungsindexes des Auges oder auch der Glasplatte liegt - was zum Beispiel für Wasser oder Kochsalzlösung der Fall ist - wird die Brechung des Laserlichts durch die Oberfläche der transparenten Platte bestimmt und nicht mehr durch die Hornhautoberfläche. Die Oberfläche der transparenten Platte kann definiert gestaltet werden, so dass das Verhalten der Phasenfront des Laserstrahls definiert ist. Im einfachsten Fall weist die Platte eine nach außen hin (also auf der dem Aufnahmeraum abgewandten Seite) ebene Oberfläche auf, so dass es nicht zu einer Brechung kommt, wenn der Laserstrahl senkrecht auf der transparenten Platte auftrifft. Der Laserstrahl durchläuft die transparente Platte, die Flüssigkeit und die Homhautoberfläche, wobei es beim Übergang von der transparenten Platte zur Flüssigkeit und beim Übergang von der Flüssigkeit zur Hornhautoberfläche zu praktisch keiner Brechung und somit zu praktisch keiner Verformung der Phasenfront kommt.

Das Material der Wahl für die Platte wird hauptsächlich Glas sein, weil es kratzfest und stabil ist sowie einen geeigneten Brechungsindex hat.

Naturgemäß muss vermieden werden, dass die ebene Oberfläche der Platte schief zum Laserstrahl steht, denn dann wird der Effekt der ebenen Oberfläche wenigstens zum Teil wieder unterlaufen. Um ein genaues Positionieren der Vorrichtung auf dem Auge zu ermöglichen, kann die Vorrichtung eine geeignete Markierung aufweisen. Bevorzugt befindet sich die Markierung auf der Platte. Es kann sich hierbei um eine kreisförmige Markierung handeln, z.B. in der Größe der Iris oder einer typischen Pupillengröße, die so gestaltet ist, dass die Vorrichtung dann ideal auf dem Auge aufsitzt, wenn sich die kreisförmige Markierung mit einer Struktur im Auge, also eben beispielsweise der Iris oder der Pupille, deckt (oder mit ihr konzentrisch ist).

Grundsätzlich kann die Vorrichtung eine beliebige Form haben. Bei einem Auge bietet sich die Ringform des Grundkörpers jedoch an. Vorzugsweise weist der Grundkörper eine innere Ringwand und eine äußere Ringwand auf. Wenn der Grundkörper elastisch sein soll, wenn er aufs Auge aufgesetzt wird, muss er aus Gummi ausgebildet sein. Er kann aber auch unelastisch sein, z.B. aus chirurgischem Stahl bestehen. Dann muss sich das Auge anpassen. Letzteres hat den Vorteil, dass die Vorrichtung besonders stabil ist.

Es genügt grundsätzlich ein einziger Einlass für das Einbringen von Flüssigkeit, einfacher gestaltet sich das Verbringen von Flüssigkeit in den Aufnahmeraum jedoch, wenn der Grundkörper eine zweite Öffnung aufweist, also einen Auslass für von der Flüssigkeit verdrängte Luft und für überschüssige Flüssigkeit.

Die Vorrichtung hält durch Unterdruck an der Hornhaut. Dieser Unterdruck kann durch Absaugen mit einer geeigneten Vorrichtung gebildet sein. Der Unterdruck muss in dem Fußbereich des Grundkörpers ausgebildet sein, und zwar unabhängig von der Flüssigkeit in dem Aufnahmeraum. Daher ist bevorzugt in dem Fußbereich des Grundkörpers ein gegenüber dem Aufnahmeraum bei auf das Auge aufgesetzter Vorrichtung dicht abgetrennter Saugraum ausgebildet, und es ist eine Absaugöffnung zum Absaugen von Luft aus dem Saugraum vorgesehen. Naturgemäß muss die Absaugöffnung bei auf das Auge aufgesetzter Vorrichtung von außen zugänglich sein, damit z.B. eine Pumpe angeschlossen werden kann.

Die erfindungsgemäße Vorrichtung kann Teil eines bei einem Eingriff am Auge einsetzbaren Systems sein, zu dem ein mit einer Flüssigkeit (wie beispielsweise Wasser oder Kochsalzlösung) gefüllter Vorratsbehälter gehört, und wobei das System ferner eine Pumpe aufweist, die mit Einlass und Auslass des Grundkörpers verbunden ist und es ermöglicht, die Flüssigkeit bei auf ein Auge aufgesetzter Vorrichtung in den Aufnahmeraum zu pumpen, z.B. zu saugen, oder beständig durch den Aufnahmeraum hindurch fließen zu lassen.

Die Vorrichtung kann auch Teil eines Systems sein, zu dem eine Pumpe gehört, die mit der Absaugöffnung verbunden ist, um einen Unterdruck in dem Saugraum auszubilden. Diese Pumpe unterscheidet sich von der oben genannten Flüssigkeitspumpe.

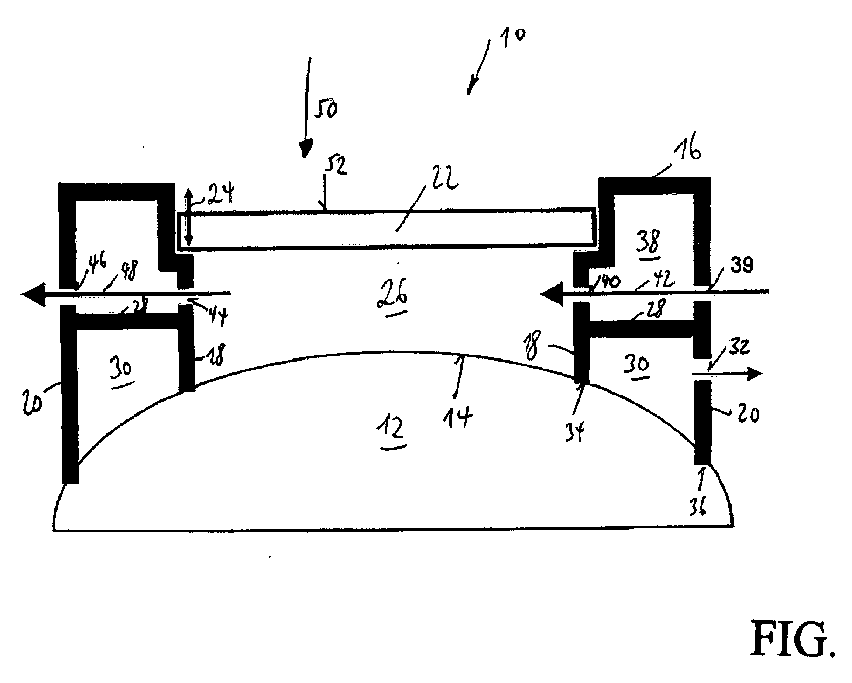

Nachfolgend wird eine bevorzugte Ausführungsform der Erfindung unter Bezug auf die Zeichnung beschrieben, wobei die einzige Figur schematisch einen Querschnitt durch die erfindungsgemäße Vorrichtung veranschaulicht, welche auf einem Auge sitzt.

Die Figur zeigt eine im ganzen mit 10 bezeichnete erfindungsgemäße Vorrichtung, die auf ein Auge 12, insbesondere auf die Hornhautoberfläche 14 des Auges 12, aufgesetzt ist. Die Vorrichtung 10 umfasst einen Grundkörper 16, der vorliegend als ringförmig gedacht werden soll und eine innere Ringwand 18 sowie eine äußere Ringwand 20 umfasst. Wie der Figur zu entnehmen ist, ist die innere Ringwand 18 in ihrem Fußbereich kürzer als die äußere Ringwand 20, so dass der Grundkörper 16 passgenau auf die Oberfläche 14 des Auges 12 aufgesetzt werden kann. Der Grundkörper 16 trägt eine Glasplatte 22, die höhenverstellbar ist (vgl. den Doppelpfeil 24). Der genaue Mechanismus der Höhenverstellung wird vorliegend nicht erläutert, da eine Vielzahl von Möglichkeiten bestehen. Gewährleistet muss sein, dass die Glasplatte 22 zum Grundkörper 16 hin abgedichtet ist: Bei auf das Auge 12 aufgesetzter Vorrichtung 10 soll ein Aufnahmeraum 26 zwischen der Hornhautoberfläche 14 und der Glasplatte 22 gebildet sein.

Das Aufsetzen der Vorrichtung 10 auf das Auge 12 erfolgt mit Hilfe von Unterdruck. Um dies zu ermöglichen, sind die innere Ringwand 18 und die äußere Ringwand 20 durch eine Trennwand 28 miteinander verbunden, durch die bei auf das Auge aufgesetzter Vorrichtung 10 ein gegenüber dem Aufnahmeraum 26 abgedichteter Saugraum 30 ausgebildet ist. Durch eine Öffnung 32 kann mittels einer in der Figur nicht gezeigten Pumpe die Luft aus dem Saugraum 30 abgesogen werden. Dadurch werden der Fußbereich 34 des Innenrings 18 und der Fußbereich 36 des Innenrings 20 gegen die Homhautoberfläche 14 gedrückt. Es ist vorteilhaft, wenn zumindest der Fußbereich 34 und der Fußbereich 36 eine gewisse Elastizität aufweisen. Dies ist auf jeden Fall dann gegeben, wenn der gesamte Grundkörper 16 aus Gummi besteht. Es ist aber nicht ausgeschlossen, dass der Grundkörper aus chirurgischem Stahl besteht. Dann ist die Vorrichtung stabil, wobei sich das Auge an sie anpassen kann. Nachdem nun die Vorrichtung 10 auf die Homhautoberfläche 14 des Auges aufgesetzt ist, wird Flüssigkeit, bevorzugt Wasser oder Kochsalzlösung, in den Aufnahmeraum 26 hineingebracht. Die Flüssigkeit wird von außen über einen Einlass 39 an der Außenringwand in einen von dem Saugraum 30 durch die Trennwand 28 getrennten Aufnahmeraum 38 eingebracht und gelangt durch einen Einlass 40 in der Innenringwand entsprechend dem Pfeil 42 in den Aufnahmeraum 26. Die Flüssigkeit kann über einen Auslass 44 in der Innenringwand 18 und einen Auslass 46 in der Außenringwand 20 entsprechend dem Pfeil 48 den Aufnahmeraum 26 verlassen. Es ist möglich, mit Hilfe einer Pumpe den Aufnahmeraum 26 einmal zu füllen. Da sich bei Laserbestrahlung Blasen bilden können, können diese von der Hornhautoberfläche dadurch wegtransportiert werden, dass die Flüssigkeit ständig bewegt wird, sie also über die Einlässe 39 und 40 durch den Aufnahmeraum 26 und durch die Auslässe 44 sowie 46 beständig fließt.

Es sei darauf hingewiesen, dass die Figur die Einlässe 39, 40 und die Auslässe 44, 46 sämtlich zeigt. Dies muss nicht dem realen Querschnitt der Vorrichtung entsprechen. Bei der realen Vorrichtung müssen die Einlässe 40 und 39 nicht fluchten, der Auslass 44 muss auch nicht mit den Einlässen 40 und 39 fluchten, und die Auslässe 44 und 46 müssen ebenfalls nicht fluchten.

Die Vorrichtung 10 bewirkt nun folgendes: während ein ohne die Vorrichtung 10 direkt auf der Homhautoberfläche 14 des Auges 12 auftreffender Laserstrahl wegen der Krümmung der Hornhaut gebrochen wird, so dass die Fokussierung des Laserstrahls erschwert ist, trifft ein in der Figur symbolisch mit 50 bezeichneter Laserstrahl bei Verwendung der Vorrichtung 10 auf einer ebenen Oberfläche 52 der Glasplatte 22 auf und wird nicht gebrochen. Beim Übergang von der Glasplatte 22 zu der in dem Aufnahmeraum 26 befindlichen Flüssigkeit kommt es zu praktisch keiner Brechung, und beim Übergang von der Flüssigkeit in dem Aufnahmeraum 26 zur Hornhautoberfläche 14 kommt es auch zu praktisch keiner Brechung. Dadurch kann der Laserstrahl 50 definiert auf eine bestimmte Tiefe in dem Auge 12 fokussiert werden. Die Vorrichtung ist somit ein wertvolles Hilfsmittel bei der Durchführung von refraktiv-chirurgischen Eingriffen mit Hilfe von Laserstrahlen.

## Patentansprüche

1. Auf ein Auge (12) aufsetzbare Vorrichtung (10) mit einem Grundkörper (16), der eine für einen vorbestimmten Wellenlängenbereich transparente Platte (22) trägt und einen Fußbereich (34, 36) aufweist, welcher ein Aufsetzen der Vorrichtung auf die Hornhautoberfläche (14) eines Auges (12) ermöglicht derart, dass dann zwischen der transparenten Platte (22) und der Hornhautoberfläche (14) ein Aufnahmeraum (26) für eine Flüssigkeit ausgebildet ist, und wobei an dem Grundkörper (16) zumindest ein Einlass (39, 40) für das Einbringen von Flüssigkeit in den Aufnahmeraum (26) bereitgestellt ist,
**dadurch gekennzeichnet,**
**dass** zur Steuerung der Flüssigkeitsmenge in dem Aufnahmeraum (26) die Platte (22) in variable Stellungen verbringbar (24) ist, denen ein unterschiedlich großer Aufnahmeraum (26) entspricht.

2. Vorrichtung nach Anspruch 1,
**dadurch gekennzeichnet,**
**dass** die Platte (22) eine nach außen hin ebene Oberfläche (52) hat.

3. Vorrichtung nach Anspruch 1 oder 2,
**dadurch gekennzeichnet,**
**dass** die Platte (22) aus Glas ausgebildet ist.

4. Vorrichtung nach einem der vorhergehenden Ansprüche,
**dadurch gekennzeichnet,**
**dass** die Platte (22) eine Markierung aufweist.

5. Vorrichtung nach einem der vorhergehenden Ansprüche,
**dadurch gekennzeichnet,**
**dass** der Grundkörper (16) ringförmig ist und vorzugsweise eine innere Ringwand (18) und eine äußere Ringwand (20) aufweist.

6. Vorrichtung nach einem der vorhergehenden Ansprüche,
**dadurch gekennzeichnet,**
**dass** der Grundkörper (16) aus chirurgischem Stahl gebildet ist.

7. Vorrichtung nach einem der vorhergehenden Ansprüche,
**dadurch gekennzeichnet,**
**dass** an dem Grundkörper (16) auch ein Auslass (44, 46) für Flüssigkeit bereitgestellt ist.

8. Vorrichtung nach einem der vorhergehenden Ansprüche,
**dadurch gekennzeichnet,**
**dass** in dem Fußbereich (34, 36) des Grundkörpers ein gegenüber dem Aufnahmeraum (26) bei auf das Auge (12) aufgesetzter Vorrichtung (10) dicht abgetrennter Saugraum (30) ausgebildet ist, der eine bei auf das Auge (12) aufgesetzter Vorrichtung (10) zugängliche Absaugöffnung (32) aufweist.

9. Bei einem Eingriff am Auge einsetzbares System mit einer Vorrichtung (10) nach Anspruch 7, mit
einem mit einer Flüssigkeit gefüllten Vorratsbehälter, und mit
einer Pumpe, die mit dem Einlass (36) und dem Auslass (46) des Grundkörpers (16) verbunden ist und es ermöglicht, die Flüssigkeit bei auf ein Auge (12) aufgesetzter Vorrichtung (10) in oder durch den Aufnahmeraum (26) zu transportieren.

10. Bei einem Eingriff am Auge einsetzbares System gemäß Patentanspruch 9, wobei die Vorrichtung (10) zusätzlich die Merkmale des Anspruchs 8 beinhaltet und mit
einer zusätzlichen Pumpe, die mit der Absaugöffnung (32) verbunden ist, um einen Unterdruck in dem Saugraum (30) auszubilden.

## Claims

1. Device (10) being able to be fitted onto an eye (12), including a base body (16) supporting a plate (22) transparent to a predetermined wavelength range and having a foot area (34, 36), which allows fitting the device onto the cornea surface (14) of an eye (12) such that a receiving space (26) for a liquid is then formed between the transparent plate (22) and the cornea surface (14), and wherein at least one inlet (39, 40) for introducing liquid into the receiving space (26) is provided on the base body (16),
**characterized in that**
for controlling the amount of liquid in the receiving space (26), the plate (22) can be brought into variable positions (24), to which a differently sized receiving space (26) corresponds.

2. Device according to claim 1,
**characterized in that**
the plate (22) has a surface (52) flat towards the outside.

3. Device according to claim 1 or 2,
**characterized in that**
the plate (22) is formed of glass.

4. Device according to any one of the preceding claims,
**characterized in that**
the plate (22) has a mark.

5. Device according to any one of the preceding claims,
**characterized in that**
the base body (16) is annular and preferably has an interior annular wall (18) and an exterior annular wall (20).

6. Device according to any one of the preceding claims,
**characterized in that**
the base body (16) is formed of surgical steel.

7. Device according to any one of the preceding claims,
**characterized in that**
an outlet (44, 46) for liquid is also provided on the base body (16).

8. Device according to any one of the preceding claims,
**characterized in that**
in the foot area (34, 36) of the base body, a suction space (30) tightly separated with respect to the receiving space (26) is formed with the device (10) fitted onto the eye (12), which has a suction opening (32) accessible with the device (10) fitted onto the eye (12).

9. System employable in a procedure on the eye including a device (10) according to claim 7, comprising
a reservoir filled with a liquid, and comprising
a pump connected to the inlet (36) and the outlet (46) of the base body (16) and allowing transporting the liquid into or through the receiving space (26) with the device (10) fitted onto an eye (12).

10. System employable in a procedure on the eye according to claim 9, wherein the device (10) additionally includes the features of claim 8, and including an additional pump, which is connected to the suction opening (32) in order to form a negative pressure in the suction space (30).

## Revendications

1. Dispositif (10) pouvant être placé sur un oeil (12), avec un corps (16) de base, qui porte une plaque transparente (22) pour une plage prédéfinie de longueurs d'ondes et présente une zone (34, 36) de pied, laquelle permet un placement du dispositif sur la surface (14) de la cornée d'un oeil (12), de telle sorte qu'un espace (26) de logement pour un liquide est constitué entre la plaque transparente (22) et la surface (14) de la cornée et moyennant quoi au moins une entrée (39, 40) est préparée sur le corps (16) de base, pour l'apport de liquide dans l'espace (26) de logement,
**caractérisé en ce**
**que** la plaque (22) peut être amenée dans des positions variables (24), pour piloter la quantité de liquide dans l'espace (26) de logement, positions auxquelles correspond un espace (26) de logement de dimension différente.

2. Dispositif selon la revendication 1,
**caractérisé en ce**
**que** la plaque (22) a une surface (52) plane vers l'extérieur.

3. Dispositif selon la revendication 1 ou 2,
**caractérisé en ce**
**que** la plaque (22) est constituée de verre.

4. Dispositif selon l'une quelconques des revendications précédentes,
**caractérisé en ce**
**que** la plaque (22) présente un marquage.

5. Dispositif selon l'une quelconques des revendications précédentes,
**caractérisé en ce**
**que** le corps (16) de base est de forme annulaire et présente de préférence une paroi annulaire intérieure (18) et une paroi annulaire extérieure (20).

6. Dispositif selon l'une quelconques des revendications précédentes,
**caractérisé en ce**
**que** le corps (16) de base est formé en acier chirurgical.

7. Dispositif selon l'une quelconques des revendications précédentes,
**caractérisé en ce**
**qu'**une sortie (44, 46) est aussi préparée pour le liquide sur le corps (16) de base.

8. Dispositif selon l'une quelconques des revendications précédentes,
**caractérisé en ce**
**que**, dans la zone (34, 36) de pied du corps de base, un espace (30) d'aspiration, séparé hermétiquement, est constitué, en face de l'espace (26) de logement, dans le cas d'un dispositif (10), placé sur l'oeil (12), qui présente une ouverture (32) d'aspiration, accessible, dans le cas d'un dispositif (10), placé sur l'oeil (12).

9. Système, pouvant être mis en place en cas d'intervention sur l'oeil, avec un dispositif (10), selon la revendication 7, avec
un réservoir de stockage, rempli de liquide et avec
une pompe, qui est reliée à l'entrée (36) et à la sortie (46) du corps (16) de base et qui permet de transporter le liquide dans ou à travers l'espace (26) de logement, dans le cas d'un dispositif (10), placé sur un oeil (12).

10. Système, pouvant être mis en place en cas d'intervention sur l'oeil, conformément à la revendication 9, moyennant quoi le dispositif (10) présente, en plus, les caractéristiques de la revendication 8 et avec
une pompe supplémentaire, qui est reliée à l'ouverture (32) d'aspiration, pour constituer une dépression dans l'espace (30) d'aspiration.
